# EUROPÄISCHE PATENTSCHRIFT

(11) **EP 0 762 858 B1**
(45) Veröffentlichungstag und Bekanntmachung des Hinweises auf die Patenterteilung: **06.09.2000**
(21) Anmeldenummer: 95923215.8
(22) Anmeldetag: 31.05.1995
(51) Int. Cl.: A61F 5/01, A61F 13/08

(54) **SCHLAUCHFÖRMIGE BANDAGE FÜR MENSCHLICHE KÖRPERTEILE**
TUBULAR BANDAGE FOR PARTS OF THE HUMAN BODY
BANDAGE TUBULAIRE POUR PARTIES DU CORPS HUMAIN

(30) Priorität: 01.06.1994 DE 4419260
(43) Veröffentlichungstag der Anmeldung: 19.03.1997
(73) Patentinhaber: Bauerfeind GmbH & Co., D-47906 Kempen (DE)
(72) Erfinder: BAUERFEIND, Hans, B., D-47906 Kempen (DE); REINHARDT, Holger, D-47906 Kempen (DE)
(74) Vertreter: Bardehle, Heinz, Dipl.-Ing.
(86) Internationale Anmeldenummer: EP9502064
(87) Internationale Veröffentlichungsnummer: WO9532693

(56) Entgegenhaltungen:
- WO-A-88/00819
- DE-A- 3 511 250
- DE-C- 664 711
- DE-U- 9 203 328
- GB-A- 2 019 726
- GB-A- 2 241 647
- US-A- 4 013 070

## Beschreibung

Die Erfindung bezieht sich auf eine schlauchförmige Bandage für menschliche Körperteile mit elastischen und weniger elastischen Teilen. Eine derartige Bandage ist beispielsweise in der DE-PS 34 41 496 beschrieben. Es handelt sich dabei um eine Sprunggelenkbandage in Form einer Socke aus dehnbarem Gewebe, auf das ein Streifen aus nichtdehnbarem Material, z.B. aus Leder aufgenäht ist. Der Lederstreifen erstreckt sich dabei vom oberen Ende der Socke bis in die Nähe der Fußsohle. Der Lederstreifen dient dazu, einen dünnen Stab aus federndem Material aufzunehmen. Schräg über den Lederstreifen ist ein Band gelegt und mit der Socke vernäht, das die eine Seite eines Klettverschlusses für ein aus einem Gummizug bestehendes Umschlingungsband bildet, das über den Spann des Fußes gelegt werden kann. Das schräg über den Lederstreifen gelegte Band übt keinerlei Wirkung auf den Lederstreifen aus, es dient lediglich zur Festlegung des Umschlingungsbandes.

Es ist darüberhinaus bekannt, schlauchförmige Bandagen, die insgesamt aus elastischem Material bestehen, zur Beeinflussung des Sitzes der Bandage mit Klettbändern zu versehen (US-PS 40 36 220, DE-GBM 92 11 728, DE-GBM 90 17 450). Außerdem gehören elastische Bandagen zum Stand der Technik, die aus einem Streifen aus elastischem Material zusammengelegt und an der Stoßstelle bzw. Überlappungsstelle durch Klettbänder zusammengehalten werden (DE-OS 42 30 165, US-PS 38 56 008).

Schließlich sei noch auf die DE-A 35 11 250 verwiesen, in der eine Kniegelenk-Bandage beschrieben ist. Bei einer Ausführung dieser insgesamt aus elastischem Material bestehenden Kniegelenk-Bandage sind auf diese beidseitig Taschen aufgenäht, in denen Stäbe aus Aluminiumblech untergebracht sind, die in Höhe des Kniegelenks gelenkig miteinander verbunden sind. Die Kniegelenk-Bandage ist außerdem mit Klettverschluß-Bändern versehen, durch die die Elastizität der Bandage allerdings nicht aufgehoben wird.

Eine grundsätzlich anders gestaltete Bandage geht aus der US-PS 4 013 070 hervor, die nicht schlauchförmig, sondern flach ausgebildet ist, wobei ein elastisches Materialstück auch bei Anlegen von Bändern seine elastische Wirkung beibehält.

Der Erfindung liegt die Aufgabe zugrunde, das Anziehen schlauchförmiger Bandagen für menschliche Körperteile zu erleichtern, ohne daß dabei deren therapeutische Wirkung verloren geht. Ausgehend von einer Bandage mit elastischen und wesentlich weniger elastischen Teilen ist die erfindungsgemäße Bandage dadurch gekennzeichnet, daß mindestens ein den elastischen Teil der Bandage bildender Längsstreifen in die Bandage eingesetzt ist, die neben dem Längsstreifen aus dem wesentlich weniger elastischen bzw. unelastischen Material besteht, und die elastischen Teile durch am unelastischen Teil befestigte Bänder von der Längsrichtung wesentlich geringerer elastischer Längsdehnungsfähigkeit bzw. Unelastizität derart überbrückbar sind, daß durch Festlegung der Bänder am unelastischen Teil der Bandage die Elastizität der elastischen Teile wesentlich verringert bzw. aufgehoben werden kann.

Eine andere, vor allem für das Knie günstig verwendbare Bandage enthält zwei im wesentlichen gleiche elastische Teile, von denen das eine sich vom einen Rand der Bandage bis etwa zu deren Mitte und das andere Teil anschließend daran bis zum anderen Rand der Bandage erstreckt, wobei jedes Teil in Umfangsrichtung im wesentlichen den halben Umfang überdeckt und beide Teile um 180° gegeneinander versetzt sind, und die elastischen Teile durch am unelastischen Teil befestigte Bänder von in Längsrichtung wesentlich geringerer elastischer Längsdehnungsfähigkeit bzw. Unelastizität derart überbrückbar sind, daß durch Festlegung der Bänder am unelastischen Teil der Bandage die Elastizität der elastischen Teile wesentlich verringert bzw. aufgehoben werden kann.

Durch die in die Bandage eingebrachten elastischen Teile wird zunächst das Anziehen der Bandage wesentlich erleichtert, da sich die elastischen Teile dabei soweit ausdehnen können, daß ein Überziehen der Bandage z.B. über das Sprunggelenk oder das Kniegelenk leicht möglich ist. Nach dem Anziehen der Bandage wird dieser dann die für die Erzielung einer therapeutischen Wirkung unerwünschte Elastizität dadurch genommen, daß die die elastischen Teile überbrückenden Bänder an der Bandage festgelegt werden, wodurch ein Zug auf die elastischen Teile durch die diese überbrückenden Bänder vollständig aufgefangen wird. Die Bandage verliert damit ihre Elastizität und wird je nach Gestaltung der Bänder praktisch unelastisch, so daß beim Tragen der Bandage deren praktische Unelastizität sich auf das jeweilige Körperteil voll auswirken kann. Eine solche Unelastizität ist z.B. dann erwünscht, wenn es sich um die Immobilisierung oder Stützung von Gliedmaßen handelt.

Die im wesentlichen zwei gleiche elastische Teile enthaltende Bandage kann man dadurch zweckmäßig weiterbilden, daß im Bereich der in Längsrichtung der Bandage verlaufenden Verbindungen der elastischen und der weniger elastischen Teile in die Bandage jeweils eine starre Längsschiene mit einem Scharnier eingebaut ist, deren Achse etwa mit der Gelenkachse zusammenfällt. Eine solche Bandage läßt sich leicht über das Kniegelenk ziehen und danach an diesem durch die Bänder festlegen, wobei durch die Schienen mit dem Scharnier dafür gesorgt wird, daß das Kniegelenk nur in einer Richtung, nämlich um die Scharnierachse, abknickbar ist.

Zweckmäßig bringt man für die Festlegung der Bänder an diesen Klettverschlüsse an. Die Bänder kann man dabei durch Umlenkringe ziehen, die an der Bandage befestigt sind, womit sich das Anspannen der Bänder bequem durchführen läßt.

Ausführungsbeispiele der Erfindung sind in den Figuren dargestellt und es zeigen
- Figur 1: eine Bandage mit einem im wesentlichen unelastischen Längsstreifen,
- Figur 2: eine Bandage mit gegeneinander versetzten elastischen Teilen.

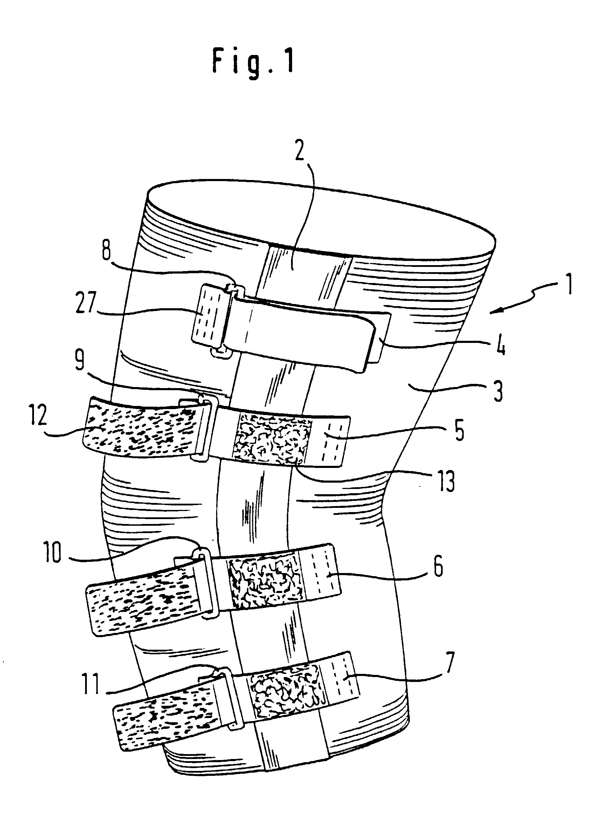

Figur 1 zeigt die schlauchförmig ausgebildete Bandage 1, die bis auf den Längsstreifen 2 als ihr eines Teil 3 im wesentlichen unelastisch ausgebildet ist. Das Teil 3 besteht nämlich aus einem nichtdehnbarem Gewebe. In das Teil 3 ist der Längsstreifen 2 eingesetzt, insbesondere eingewebt oder eingewirkt, der aus einem elastischen Material besteht. Dieser Längsstreifen 2 ermöglicht es der Bandage 1 beim Überziehen über ein Gelenk, z.B. das Kniegelenk, daß sich die Bandage 1 im Bereich des Streifens 2 dehnt und daher mühelos über das betreffende Gelenk gezogen werden kann. Der Längsstreifen 2, der also den elastischen Teil der Bandage 1 bildet, ist durch die im wesentlichen unelastischen Bänder 4, 5, 6 und 7 überbrückt, die auf ihrer in der Figur 1 rechten Seite an dem unelastischen Teil 3 befestigt, insbesondere angenäht sind. Auf der anderen Seite der Bänder 4 bis 7 besitzen diese jeweils einen Ring 8, 9 ,10 und 11, der seinerseits in bekannter Weise mittels einer Schlaufe 27 an dem unelastischen Teil 3 befestigt ist. Die Schlaufe ist hier an dem unelastischen Teil angenäht. Um dem elastischen Längsstreifen 2 im Effekt die Elastizität zu nehmen, werden die Bänder 4-7 durch die Ringe 8-11 gezogen und unter Strammziehen mittels jeweils eines Klettverschlusses 12/13 hinsichtlich ihrer wirksamen Länge festgelegt. Damit ist der elastische Längsstreifen 2 durch die unelastischen Bänder 4-7 überbrückt und kann sich nicht mehr ausdehnen.

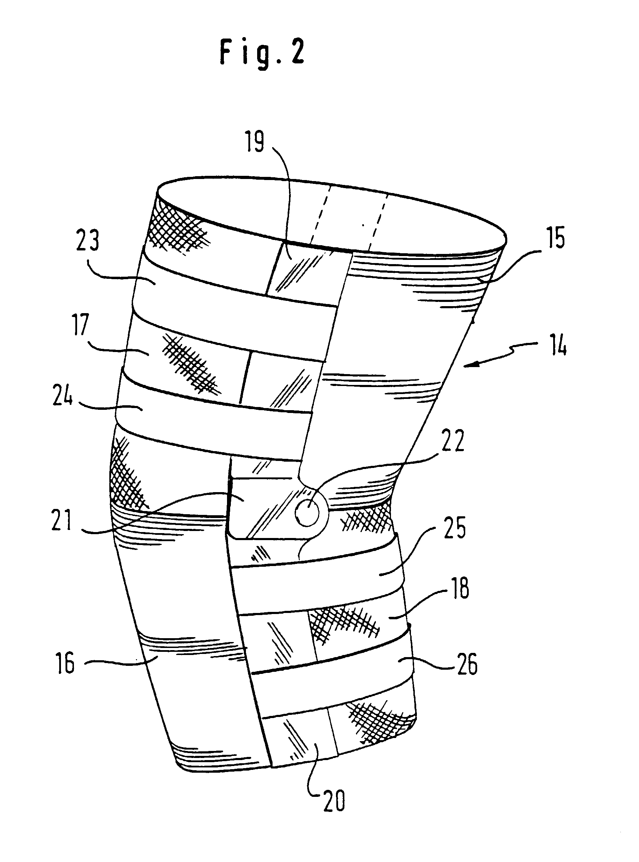

In der Figur 2 ist eine Variante dargestellt, bei der die Bandage 14 die beiden unelastischen Teile 15 und 16 und die elastischen Teile 17 und 18 enthält. Die unelastischen Teile 15 und 16 sind gewissermaßen diagonal angeordnet, was auch für die elastischen Teile 17 und 18 gilt. Im Bereich des Zusammenstoßens der unelastischen Teile 15/16 mit den elastischen Teilen 17/18 sind Längsschienen 19/20 in die Bandage 14 eingebaut, z.B. mittels eines einhüllenden Gewebes, das auf das Material der Bandage 14 aufgenäht ist. Diese Längsschienen 19 und 20 enthalten jeweils ein Scharnier 21 mit der Achse 22, die etwa im Bereich der Gelenkachse liegt und bei Anziehen der Bandage 14 über das Kniegelenk diesem um die Gelenkachse die Beweglichkeit erhält. Bei der in Figur 2 dargestellten Bandage 14 sind auf beiden Seiten der Bandage derartige Längsschienen 19 und 20 vorgesehen, die hintere Längsschiene ist jedoch nicht sichtbar und nur durch gestrichelte Linien angedeutet.

Die elastischen Teile 17 und 18 der Bandage 14 sind durch die unelastischen Bänder 23 und 24 sowie 25 und 26 überbrückt, die in der gleichen Weise wie die Bänder 4-7 gemäß Figur 1 den zugehörigen unelastischen Teil 17 bzw. 18 überbrücken und damit diesem die Elastizität nehmen. Die Befestigung der Bänder 23/24 und 25/26 erfolgt dann in der gleichen Weise, wie dies im Zusammenhang mit der Figur 1 dargestellt ist.

## Patentansprüche

1. Schlauchförmige Bandage (1) für menschliche Körperteile mit elastischen und wesentlich weniger elastischen Teilen (2, 3), **dadurch gekennzeichnet**, daß mindestens ein den elastischen Teil (2) der Bandage bildender Längsstreifen (2) in die Bandage (1) eingesetzt ist, die neben dem Längsstreifen (2) aus dem wesentlich weniger elastischen bzw. unelastischen Material (3) besteht, und die elastischen Teile (2) durch am unelastischen Teil (3) befestigte Bänder (4, 5, 6, 7) von in Längsrichtung wesentlich geringerer elastischer Längsdehnungsfähigkeit bzw. Unelastizität derart überbrückbar sind, daß durch Festlegung der Bänder (4, 5, 6, 7) am unelastischen Teil (3) der Bandage (1) die Elastizität der elastischen Teile (2) wesentlich verringert bzw. aufgehoben werden kann.

2. Schlauchförmige Bandage (1) für menschliche Körperteile mit elastischen und wesentlich weniger elastischen Teilen (2, 3), **dadurch gekennzeichnet,** daß sie zwei im wesentlichen gleiche elastische Teile (17, 18) enthält, von denen das eine sich vom einen Rand der Bandage (14) bis etwa zu deren Mitte und das andere Teil anschließend daran bis zum anderen Rand der Bandage (14) erstreckt, wobei jedes Teil (17, 18) in Umfangsrichtung im wesentlichen den halben Umfang überdeckt und beide Teile (17, 18) um 180° gegeneinander versetzt sind, und die elastischen Teile (2) durch am unelastischen Teil (3) befestigte Bänder (4, 5, 6, 7) von in Längsrichtung wesentlich geringerer elastischer Längsdehnungsfähigkeit bzw. Unelastizität derart überbrückbar sind, daß durch Festlegung der Bänder (4, 5, 6, 7) am unelastischen Teil (3) der Bandage (1) die Elastizität der elastischen Teile (2) wesentlich verringert bzw. aufgehoben werden kann.

3. Bandage nach Anspruch 2 für Gelenke, dadurch gekennzeichnet, daß im Bereich der in Längsrichtung der Bandage (1) verlaufenden Verbindungen der elastischen und der weniger elastischen Teile (15,16;17,18) in die Bandage (1) jeweils eine starre Längsschiene (19,20) mit einem Scharnier (21) eingebaut ist, deren Achse (22) etwa mit der Gelenkachse zusammenfällt.

4. Bandage nach einem der Ansprüche 1 bis 3, dadurch gekennzeichnet, daß für die Festlegung der Bänder (4,5,6,7) auf diesen Klettverschlüsse (12,13) angebracht sind.

5. Bandage nach Anspruch 4, dadurch gekennzeichnet, daß die Bänder (4,5,6,7) durch Umlenkringe (8,9,10,11) gezogen sind, die an der Bandage (1) befestigt sind.

## Claims

1. Tubular bandage (1) for parts of the human body, comprising elastic and substantially less elastic parts (2, 3), characterised in that at least one longitudinal strip (2) forming the elastic part (2) of the bandage is inserted into the bandage (1), said bandage consisting of the substantially less elastic or non-elastic material (3) in addition to the longitudinal strip (2), and the elastic parts (2) can be bridged by tapes (4, 5, 6, 7) displaying substantially less elastic stretchability or non-elasticity in the longitudinal direction fastened to the non-elastic part (3) in such a manner that the elasticity of the elastic parts (2) can be substantially reduced or eliminated by fixing the tapes (4, 5, 6, 7) to the non-elastic part (3) of the bandage (1).

2. Tubular bandage (1) for parts of the human body, comprising elastic and substantially less elastic parts (2, 3), characterised in that it contains two substantially identical elastic parts (17, 18), one of which extends from one edge of the bandage (14) to approximately the centre thereof and the other of which extends from there to the other edge of the bandage (14), each part (17, 18) covering substantially half the circumference in the circumferential direction and both parts (17, 18) being offset by 180° relative to one another, and wherein the elastic parts (2) can be bridged by tapes (4, 5, 6, 7) displaying substantially less elastic stretchability or non-elasticity in the longitudinal direction fastened to the non-elastic part (3) in such a manner that the elasticity of the elastic parts (2) can be substantially reduced or eliminated by fixing the tapes (4, 5, 6, 7) to the non-elastic part (3) of the bandage (1).

3. Bandage according to claim 2 for joints, characterised in that respective rigid longitudinal splints (19, 20) with hinges (21) are incorporated into the bandage (1) in the region of the connections between the elastic and the less elastic parts (15, 16; 17, 18) extending in the longitudinal direction of the bandage (1), the axes (22) of the hinges substantially coinciding with the joint axis.

4. Bandage according to one of claims 1 to 3, characterised in that Velcro strip fasteners (12, 13) are fitted to the tapes (4, 5, 6, 7) for fixing the latter.

5. Bandage according to claim 4, characterised in that the tapes (4, 5, 6, 7) are pulled through guide rings (8, 9, 10, 11) fastened to the bandage (1).

## Revendications

1. Bandage tubulaire (1) destiné à des parties du corps humain et comportant des parties élastiques (2) et des parties nettement moins élastiques (3), caractérisé en ce qu'au moins une bande longitudinale (2) formant la partie élastique (2) du bandage est placée dans le bandage (1) qui est composé, outre de la bande longitudinale (2), du matériau nettement moins élastique ou non élastique (3) et en ce que les parties élastiques (2) sont pontées de telle sorte par des bandes (4, 5, 6, 7) fixées à la partie non élastique (3) et ayant une extensibilité longitudinale élastique nettement plus petite dans la direction longitudinale, c'est-à-dire une non-élasticité dans la direction longitudinale, que l'élasticité des parties élastiques (2) peut être nettement réduite ou supprimée par la fixation des bandes (4, 5, 6, 7) à la partie non élastique (3) du bandage (1).

2. Bandage tubulaire (1) destiné à des parties du corps humain et comportant des parties élastiques (2) et des parties nettement moins élastiques (3), caractérisé en ce qu'il contient deux parties élastiques (17, 18) qui sont globalement identiques, dont l'une s'étend d'un bord du bandage (14) environ jusqu'à son milieu et dont l'autre partie s'étend à la suite jusqu'à l'autre bord du bandage (14), chaque partie (17, 18) recouvrant en direction circonférentielle globalement la moitié de la circonférence et les deux parties (17, 18) étant décalées de 180° l'une par rapport à l'autre, et en ce que les parties élastiques (2) sont pontées de telle sorte par des bandes (4, 5, 6, 7) fixées à la partie non élastique (3) et ayant une extensibilité longitudinale élastique nettement plus petite dans la direction longitudinale, c'est-à-dire une non-élasticité dans la direction longitudinale, que l'élasticité des parties élastiques (2) peut être nettement réduite ou supprimée par la fixation des bandes (4, 5, 6, 7) à la partie non élastique (3) du bandage (1).

3. Bandage selon la revendication 2 pour des articulations, caractérisé en ce que, dans la zone des liaisons, s'étendant dans la direction longitudinale du bandage (1), des parties élastiques et moins élastiques (15, 16 ; 17, 18), il est agencé dans le bandage (1) à chaque fois une barre longitudinale rigide (19, 20) comportant une charnière (21) dont l'axe (22) coïncide sensiblement avec l'axe d'articulation.

4. Bandage selon l'une des revendications 1 à 3, caractérisé en ce que, pour la fixation des bandes (4, 5, 6, 7), des bandes agrippantes du type fermetures Velcro (12, 13) sont agencées sur ces bandes.

5. Bandage selon la revendication 4, caractérisé en ce que les bandes (4, 5, 6, 7) sont tirées par des anneaux de renvoi (8, 9, 10, 11) qui sont fixés au bandage (1).
